# EUROPEAN PATENT APPLICATION

(11) **EP 4 223 504 A1**
(43) Date of publication of application: **09.08.2023**
(21) Application number: 22155738.2
(22) Date of filing: 08.02.2022
(51) Int. Cl.: B32B 7/06, B32B 27/08, B32B 27/12, B32B 27/32, B65B 55/02, B65D 75/30, B65D 75/32, A61B 50/30, A61B 50/33, B65D 77/20

(54) **PACKAGING MATERIALS**

(71) Applicant: Danapak Flexibles A/S, 4200 Slagelse (DK)
(72) Inventor: JOHANSEN, Peter, 4200 Slagelse (DK); MØLLER, Rasmus Buch, 4200 Slagelse (DK)
(74) Representative: AWA Denmark A/S

(57) **Abstract**

The present disclosure relates to packaging materials for allowing sterilization of contents of a package comprising packaging, such as a bag or tray, manufactured from the packaging material. In further aspects, the disclosure relates to uses and methods of manufacture of such packaging materials.

## Description

The present disclosure relates to packaging materials for allowing sterilization of contents of a package comprising packaging, such as a bag or tray, manufactured from the packaging material. The disclosure also relates to uses and methods of manufacture of such packaging materials.

Such packaging materials are known in the art in various forms. Some prior art packaging materials can be used for packaging of contents, such as medical devices, which can be sterilized.

Packaging materials according to the present disclosure comprise, consist of, or essentially consist of
a porous film comprising, consisting of, or essentially consisting of a high density polyethylene,
wherein the porous film shows a microbial barrier log retention value of 3 or more according to the test method ASTM F1608 and a Bendtsen air permeability of 600 ml/min or more according to the test method ISO 5636-3, and/or wherein the high density polyethylene comprises, consists of, or essentially consists of virgin filaments, the virgin filaments being flashspun and bonded to each other using heat and pressure with essentially no binders or fillers having been added, and
a laminate comprising a heat sealing polyethylene film adhered to either an oriented polyethylene (OPE) film or to a layer of a high density polyethylene (HDPE),
wherein at least a region of said heat sealing polyethylene film has been heat sealed to at least a region of said porous film.

Surprisingly, a fully satisfactorily peelable sealing of said laminate to said porous film was obtained, avoiding fiber tears.

Advantages of such packaging materials may include one or more of recyclability, low cost, and allowing suitable sterilization of packaged contents, such as one or more medical devices. The packaging material can be manufactured as a mono-laminate, potentially essentially a mono-laminate, of polyethylene (PE), which may be recyclable as PE.

Pre-coating of the porous film with a sealing laquer may be avoided.

In particular in embodiments, wherein the packaging comprises the OPE layer, the packaging material may be a laminate or film and/or may be flexible.

The layers of the laminate may be adhered to each other by means of heat-sealing the heat sealing layer to the OPE or HDPE. Alternatively, the laminate may comprise an adhesive layer. If comprising an adhesive layer, the heat sealing layer may be adhered to the OPE or HDPE layers by means of processes including adhesive lamination, in which case the adhesive layer comprises or consists of an adhesive, and extrusion lamination, in which case the adhesive layer comprises or consists of an extrusion lamination adhesive layer, such as an adhesive layer comprising polyolefin, such as PE.

The porous film may have a thickness of 100 to 300 µm, 100 to 250 µm, 125 to 200 µm, 150 to 180 µm, 153-157 µm, or 176-180 µm.

In some embodiments, the porous film has a basis weight (ASTM D3776; EN ISO 536) of 60-90 g/m³, 70-80 g/m³, or 73-75 g/m³. Further, it may show a delamination (ASTM D2724) of 1-4 N/2.54 cm, 2-3 N/2.54 cm, or 2.2-2.4 N/2.54 cm, and/or a Gurley Hill Porosity (TAPPI T460; ISO 5636-5) of 8-36 sec/100 cm³, 20-30 sec/100 cm³, or 21-23 sec/100 cm³. The porous film may present a moisture vapour transmission rate (TAPPI T523) of 1500-1650 g/m²/24 hr, 1550-1630 g/m²/24 hr, or 1610-1620 g/m²/24 hr; and/or a hydrostatic head (AATCC TM 127; EN 20811) of 140-160 cm H₂O, 145-150 cm H₂O, or 146-148 cm H₂O; and/or a tensile strength (ASTM D5035; EN ISO 1924-2) in the machine direction orientation (MD) of 145-210 N/2.54 cm, 155-200 N/2.54 cm, or 195-197 N/2.54 cm, and in the cross direction (CD) 155-210 N/2.54 cm, 160-205 N/2.54 cm, or 199-201 N/2.54 cm; and/or an elongation (ASTM D5035; EN ISO 1924-2) in the machine direction orientation (MD) of 16-23 %, 18-22 %, or 19-21 %, and in the cross direction (CD) 19-27 %, 20-26 %, or 23-25 %; and/or an Elmendorf tear (ASTM D1424; EN 21974) in the machine direction orientation (MD) of 2.5-3.6 N, 2.6-3.5 N, or 3.2-3.4 N, and in the cross direction (CD) 3.2-3.8 N, 3.3-3.7 N, or 3.4-3.6 N; and/or a Mullen burst (ASTM D774; ISO 2758) of 850-1250 kPa, 895-1230 kPa, 1210-1220, kPa or 1212-1214 kPa; and/or a Spencer puncture (ASTM D3420) of 4850-9000 J/m², 4900-8770 J/m², 8750-8760 J/m², or 8755-8757 J/m²; and/or an opacity (TAPPI T425; ISO 2471) of 87-96 %, 88-95 %, or 90-92 %.

In embodiments, wherein the packaging comprises the OPE layer or comprises the layer of HDPE in the form of non-expanded HDPE, the packaging material may have a total thickness of 125-1000 µm, 200-800, 300-600, or 400-500 µm.

In embodiments, wherein the packaging comprises the layer of HDPE in the form of expanded HDPE, the packaging material may have a total thickness of 600-12.000 µm, 1.500-8.000 µm, or 2.000-5.000 µm.

In some embodiments, the heat sealing polyethylene film has a thickness of 15-200 µm, 30-180 µm, 50-160 µm, 70-140 µm, 90-120 µm, 50-200 µm, 100-195 µm, 150-190 µm, 170-185 µm, 15-80 µm, 30-70 µm, or 40-60 µm and is optionally a low density polyethylene film. The heat sealing polyethylene film in some embodiments may have a density (composite density) of 0.900-0.9400 g/cm³, 0.920-0.938 g/cm³, or 0.935-0.937 g/cm³. Further, it may present a tensile strength (DIN EN ISO 527-3) in the machine orientation direction (MD) of 17-23 N/mm², 18-22 N/mm², or 19-21 N/mm², and in the transverse orientation direction (TD) 15-21 N/mm², 16-20 N/mm², or 17-19 N/mm²; a strain at break (DIN EN ISO 527-3) in the machine orientation direction (MD) of 50-400 %, 100-300 %, or 150-250 %, and in the transverse orientation direction (TD) 500-900, 600-800, or 650-750 %; and a tensile stress at 10 % strain (DIN EN ISO 527-3) in both the machine and the transverse orientation direction (MD and TD) of 7-11 N/mm², 8-10 N/mm², or 8.5-9.5 N/mm². One or both sides of the heat sealing polyethylene film may have been subjected to corona treatment.

In some embodiments, the above-mentioned layer of HDPE is non-transparent and shows a haze of more than 10 %. The layer of HDPE may possess or may not possess a print on the face of the layer facing the heat sealing polyethylene film or on the face of the layer facing away from the heat sealing polyethylene film. In some embodiments, wherein the packaging material comprises said layer of HDPE in the form of a film of non-expanded HDPE, said film may present a thickness of 50-300 µm, 70-200 µm, 80-150 µm, or 90-110 µm; and/or a tensile strength (ISO 527) in the machine orientation direction (MD) of 30-300 MPa, 38-80 MPa, or 40-45 MPa, and in the transverse orientation direction (TD) 20-200 MPa, 28-70 MPa, or 30-35 MPa; and/or an elongation (ISO 527) in the machine orientation direction (MD) of 250-1500 %, 290-500 %, or 300-400 %, and in the transverse orientation direction (TD) 400-2500 %, 440-1500 %, or 450-550 %; and/or an dart impact test value (ASTM-D-1709-A) of 2-100 g/µm, 2.8-50 g/µm, or 3-30 g/µm.

In some embodiments, the oriented polyethylene film is a mono- or biaxially oriented film and has a thickness of 8-100 µm, 20-80 µm, 40-60 µm, 15-100 µm, 50-97 µm, 70-95 µm, 80-90 µm, 8-50 µm, 15-40 µm, 20-30 µm, or 24-26 µm. The oriented polyethylene film may be a film that has not been cast extruded. In some embodiments, it is non-transparent and shows a haze of more than 10 %. The oriented polyethylene film may possess or may not possess a print on the face of the film facing the heat sealing polyethylene film or on the face of the film facing away from the heat sealing polyethylene film.

In some embodiments, the oriented polyethylene film has a density of 0.900-0.970 kg/dm³, 0.910-0.925 kg/dm³, 0.930-0.960 kg/dm³, or 0.940-0.955 kg/dm³; and/or an elastic modulus in the machine direction orientation (MD) of 1300-1700 MPa, 1400-1600 MPa, or 1450-1550 MPa, and in the transverse direction orientation (TD) 1800-2200 MPa, 1900-2100 MPa, or 1950-2050 MPa; and/or a coefficient of friction of 0.30-0.40, or 0.34-0.36; and/or a water vapour transmission rate of 3-7 g/m²/24 hr, or 4-6 g/m²/24 hr.

In some embodiments, the oriented polyethylene film shows a stress at break (PN-EN ISO 527) in the machine direction (MD) of 150-170 MPa, 155-165 MPa, or 161-163 MPa, and in the transverse direction (TD) 10-20 MPa, 15-17 MPa, or 15.7-15.9 MPa; and/or a modulus of elasticity in the machine direction (MD) (ISO 527-1) of 435-475 MPa, 450-460 MPa, or 454-456 MPa, and in the transverse direction (TD) (ISO 527-2) 810-830 MPa, 813-823 MPa, or 821-823 MPa; and/or an elongation at break (PN-EN ISO 527) in the machine direction (MD) of 30-40 %, 36-40 %, or 37-39 % and in the transverse direction (TD) 6-16 %, 8-14 %, or 10-12 %; and/or a puncture resistance of 5-9 N, 6-8 N, or 7.6-7.8 N; and/or a tear strength according to the "pants" method (PN-EN ISO 6383-1) in the machine direction (MD) of 100-105 N/mm, 103.53-103.73 N/mm; or 103.62-103.64 N/mm, and in the transverse direction (TD) 270-274 N/mm, 271.73-271.93 N/mm, or 271.82-271.84 N/mm; and/or a kinetic and/or static coefficient of friction (surface inactivated; PN-ISO 8295) of 0.90 or less, or 0.7 or less.

In some embodiments, one or both sides of the oriented polyethylene film may have been subjected to corona treatment. Further, in some embodiments the face of the oriented polyethylene film facing away from the heat sealing polyethylene film may have been subjected to application of a heat resistant lacquer in order to augment the heat resistance of the oriented polyethylene film. In one embodiment, the heat resistant lacquer is an ethyl acetate, nitrocellulose-based, 2-component varnish.

In some embodiments, the adhesive layer comprises, consists of, or essentially consists of an adhesive, and wherein the heat sealing polyethylene film has been adhesive laminated to the oriented polyethylene film by means of the adhesive layer, so that the heat sealing polymer film adheres to the oriented polyethylene film.

An "adhesive", may be defined as a non-metallic substance applied to one or both surfaces of two separate items that binds them together and resists their separation. An adhesive can also be denoted "glue". The adhesive may comprise at least 20, 30, 40, 50, 60, 70, 80, 95, 98 or 99 % (w/w) of the adhesive layer.

Several types of adhesive may be usable, such as an epoxy adhesive, a polyurethane adhesive, or a polyimide adhesive.

In some further developed embodiments, a 2-component adhesive may be used for the adhesive lamination. A 2-component adhesive is typically less dependent on moisture and temperature to cure than a 1-component adhesive. The 2-component adhesive may be applied in a grammage, including hardener, of at least 1 g/m² and/or at most 20 g/m², such as 1-10 g/m², 1-7 g/m², 1-6 g/m², 1-5 g/m², 1-4 g/m², 2-3 g/m², or 2.4-2.6 g/m². The adhesive may be added to the heat sealing polyethylene film an/or the oriented polyethylene film in an amount as small as possible. Said 2-component adhesive may be the only adhesive used for the adhesive lamination. The 2-component adhesive may be a polyurethane adhesive with an isocyanate hardener. The isocyanate may be an aliphatic or, preferentially, an aromatic isocyanate.

The adhesive may require a solvent or may be solventless. When making use of a solvent, the solvent, such as e.g. ethyl acetate, and the adhesive may be mixed in a specific ratio as specified by the manufacturer.

The base and hardener of the adhesive are mixed in a ratio of 5:1 to 25:1, preferably 8:1 to 15:1, calculated on solid content. The solvent is added to provide a viscosity in the range of 13 to 30 sec, such as 15 to 22 sec, such as around 17 - 18 sec as measured by DIN CUP 4, a method well known to the skilled person under the DIN 53211 standard.

"Adhesive lamination" as the term is used herein is a well-known process of laminating films to each other. This process can involve a production line equipped with rollers to hold lengths of the heat sealing polyethylene film and the oriented polyethylene film. The adhesive is added evenly to the surface of either the heat sealing polyethylene film or the oriented polyethylene film or to both of these surfaces, after which the heat sealing polyethylene film and the oriented polyethylene film are pressed together to let the adhesive cure and produce the laminate film. The heat sealing polyethylene film and the oriented polyethylene film may be pressed together by combining or nip rolls, which may be heated, cooled or neither heated nor cooled.

In some embodiments, the laminate comprises said layer of high density polyethylene and a further adhesive layer facing the layer of high density polyethylene, and an oriented polyethylene film facing the further adhesive layer. Such a laminate may be used for trays, wherein said layer of high density polyethylene may comprise or consist of expanded or non-expanded HDPE.

In some embodiments, the laminate is in the form of a tray, optionally thermoformed, comprising a bottom wall and a number of side walls defining a spacing for an item to be packaged, and wherein the porous film is in the form of a lid for sealing to an upper edge of the tray.

In some further developed embodiments, the heat sealing polyethylene film has a thickness of 50-200 µm, 100-195 µm, 150-190 µm, 70-185 µm, and/or the oriented polyethylene film has a thickness of 25-100 µm, 50-97 µm, 70-95 µm, or 80-90 µm.

In some embodiments, the tray comprises said layer of high density polyethylene, wherein the high density polyethylene is an expanded high density polyethylene, wherein the high density polyethylene is potentially rigid. The layer of expanded high density polyethylene may have a thickness of 500-10.000 µm, 1.000-5.000 µm, or 2.000-4.000 µm.

In some further developed embodiments, the heat sealing polyethylene film has a thickness of 15-80 µm, 30-70 µm, or 40-60 µm and/or the oriented polyethylene film has a thickness of 8-50 µm, 15-40 µm, 20-30 µm, or 24-26 µm.

In embodiments, the laminate comprises the oriented polyethylene film, the heat sealing polyethylene film of the packaging material sealing to rims of the porous film, such as to constitute a packaging in the form of a bag, wherein packaged contents, such as a medical device, is potentially positioned between the porous film and the laminate, wherein the laminate is potentially a flexible laminate.

The heat sealing polyethylene film may seal all along a circumferential rim of the porous film, such as to constitute the bag, wherein the potential packaged contents, such as a medical device, positioned between the porous film and the laminate, can be packaged in a sealed manner.

Another aspect of the present disclosure provides a packaging, such as a bag or tray, for the manufacture of a package, wherein the packaging comprises, consists of, essentially consists of, or is manufactured from a packaging material according to any one of the above embodiments.

Another aspect of the present disclosure provides a package comprising the packaging, wherein contents, such as a medical device, are packed in the packaging.

Another aspect of the present disclosure provides a use of the package for manufacture of a sterilized package, such as package comprising a medical device, the package being sterilized, such as by ethylene oxide sterilization, water vapour sterilization, gamma radiation sterilization, and/or plasma sterilization.

Another aspect of the present disclosure provides a use of a laminate for manufacture of the packaging material, wherein the laminate comprises a heat sealing polyethylene film adhered to either an oriented polyethylene film or to a layer of a high density polyethylene.

In particular in embodiments, wherein the packaging comprises the OPE layer, the packaging material may be a laminate or film and/or may be flexible.

The layers of the laminate may be adhered to each other by means of heat-sealing the heat sealing layer to the OPE or HDPE. Alternatively, the laminate may comprise an adhesive layer. If comprising an adhesive layer, the heat sealing layer may be adhered to the OPE or HDPE layers by means of processes including adhesive lamination, in which case the adhesive layer comprises or consists of an adhesive, and extrusion lamination, in which case the adhesive layer comprises or consists of an extrusion lamination adhesive layer, such as an adhesive layer comprising polyolefin, such as PE.

Another aspect of the present disclosure provides a method of manufacture of a packaging material for allowing sterilization of contents of a package comprising packaging, such as a bag or tray, manufactured from the packaging material, wherein the packaging material is optionally according to any one of the embodiments disclosed herein, wherein said method comprises, consists of, or essentially consists of
providing a porous film comprising, consisting of, or essentially consisting of a high density polyethylene, wherein the porous film shows a microbial barrier log retention value of 3 or more according to the test method ASTM F1608 and a Bendtsen air permeability of 600 ml/min or more according to the test method ISO 5636-3, and/or wherein the high density polyethylene comprises, consists of, or essentially consists of virgin filaments, the virgin filaments being flashspun and bonded to each other using heat and pressure with essentially no binders or fillers having been added,
providing a laminate comprising a heat sealing polyethylene film adhered to either an oriented polyethylene film or to a layer of a high density polyethylene, such as by adhering the heat sealing polyethylene film to either the oriented polyethylene film or to the layer of the high density polyethylene, and
heat sealing at least a region of said heat sealing polyethylene film to at least a region of said porous film, such as by exposing the heat sealing polyethylene film to a sealing temperature of 80-160 °C, 85-140 °C, or 90-125 °C, for 0.5-3.0 seconds, 0.7-2.5 seconds, 0.8-2.0 seconds, or 0.9-1.0 seconds.

When producing the packaging material, the porous high density polyethyle film is superimposed on the laminate, so that the porous HDPE film contacts the heat sealing polyethylene film of the laminate at least in regions thereof. Subsequently, at the regions to be sealed, pressure is applied to the porous HDPE film and, as the case may be, either to the oriented polyethylene film or to the layer of a high density polyethylene. Heat may be applied to the porous HDPE film and/or said oriented polyethylene film or said layer of a high density polyethylene.

The method may comprise any one or more of the features and/or method steps according to any one of the above embodiments of the packaging material, the packaging, the package, and or the uses thereof according to the present disclosure.

The following detailed description discloses further non-limiting examples of embodiments with reference to the schematic drawings, in which:
Fig. 1 shows a cross-section of an embodiment of the porous film, the laminate comprising three layers, and the packaging material.
Fig. 2 is a perspective view of an embodiment of a tray manufactured from the packaging material.
Fig. 3, 4a, 4b, and 4c shows a cross-section of the embodiment of the tray shown in Fig. 2.
Figs. 5a, 5b, and 5c shows a cross-section of the embodiment of Fig. 6.
Fig. 6 is a perspective view of an embodiment of a bag manufactured from the packaging material.
Fig. 7 shows a cross-section of the embodiment of the bag shown in Fig. 6.

### Detailed description of embodiments

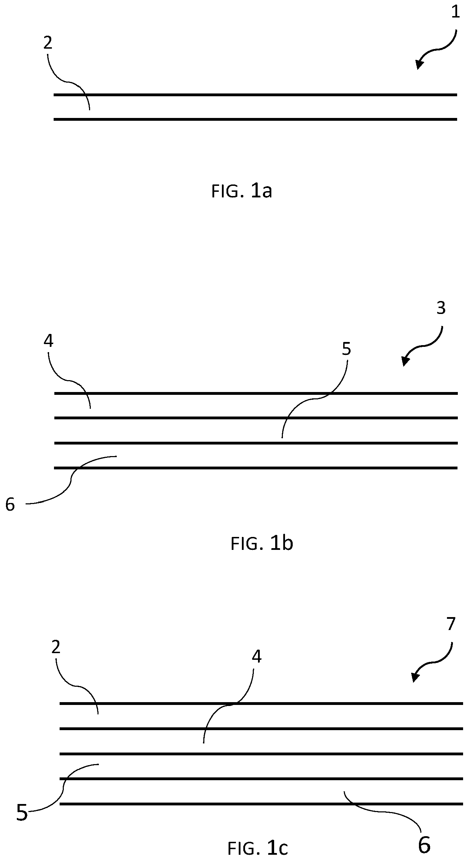

Fig. 1 shows the make-up of an embodiment of the packaging material and its constituents.

Fig. 1a shows a top layer 1 consisting of a porous HDPE film 2.

Fig. 1b shows a laminate 3 composed of a heat sealing PE film 4, an adhesive layer 5, and a layer of HDPE 6.

In Fig. 1c, the structure of the final packaging material 7, when the top layer 1 has been sealed to the laminate 3, is shown. As will appear, the heat sealing PE film 4 faces the overlying layer of porous HDPE film 2.

The laminate in the present embodiment is produced by gluing the heat sealing PE film 4 to the layer of HDPE 6 by means of an adhesive 5. The adhesive is added to both the surface of the heat sealing polyethylene film and the oriented polyethylene film, after which the heat sealing polyethylene film and the oriented polyethylene film are pressed together to let the adhesive cure and produce the laminate film. The adhesive is used in an amount of 3 g/m².

Subsequently, the packaging material is produced by superimposing the top layer 1 on the laminate 3, so that the porous HDPE film 2 contacts the heat sealing PE film 4, applying heat in the regions to be sealed at a temperature of 80 °C at the side of the porous HDPE film 2 and at a temperature of 125 °C at the side of the layer of HDPE 6 for a period of 2 seconds.

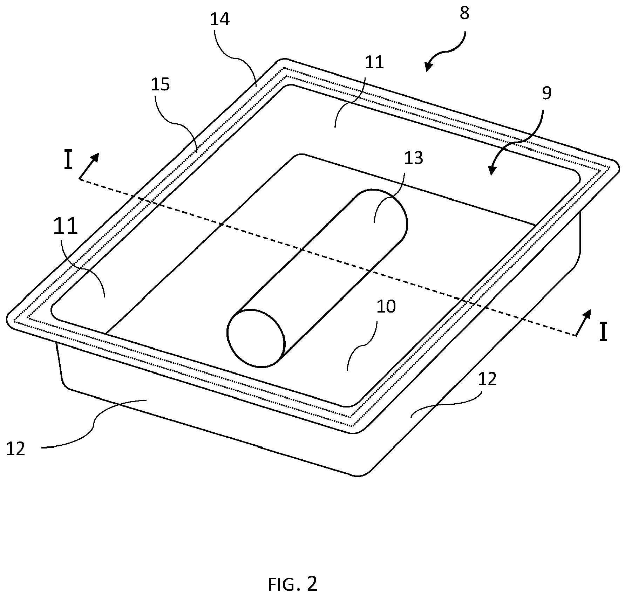

In Fig. 2, a tray 8 prepared from the packaging material is depicted. By thermoforming, a tray with a spacing 9 is provided. The tray is covered by a lid formed by a top layer 1, which in the present depictions is invisible. The tray presents a bottom wall 10, side walls 11, outer walls 12. The tray is provided with an upper edge 14 extending in a plane, so that it has been possible for the lid to be heat sealed to the edge 14 as indicated by a sealing seam 15.

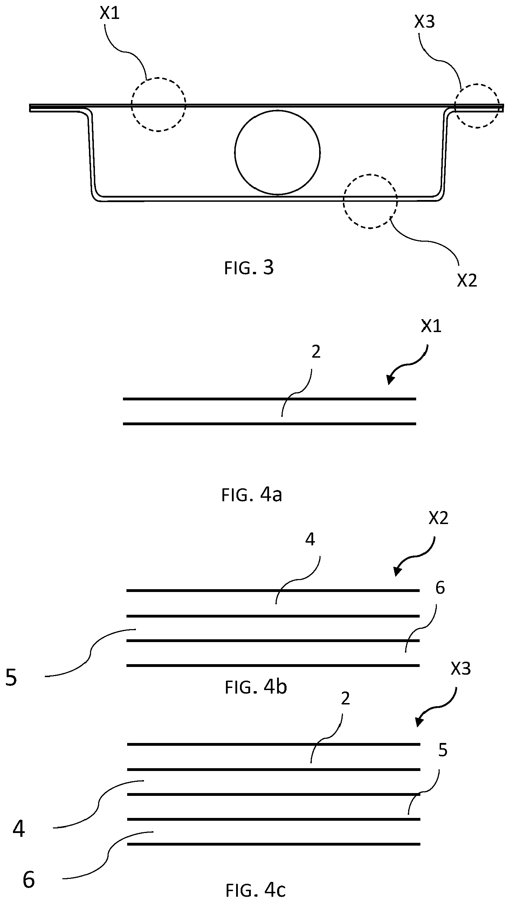

In Fig 3, a cross-section along the line II of Figure 2 is shown. The layer structure of the top layer for the lid, the laminate for the tray, and the sealed rim of the tray and the lid are shown as views X1, X2, and X3, detailed in Figs. 4a, 4b, and 4c, respectively.

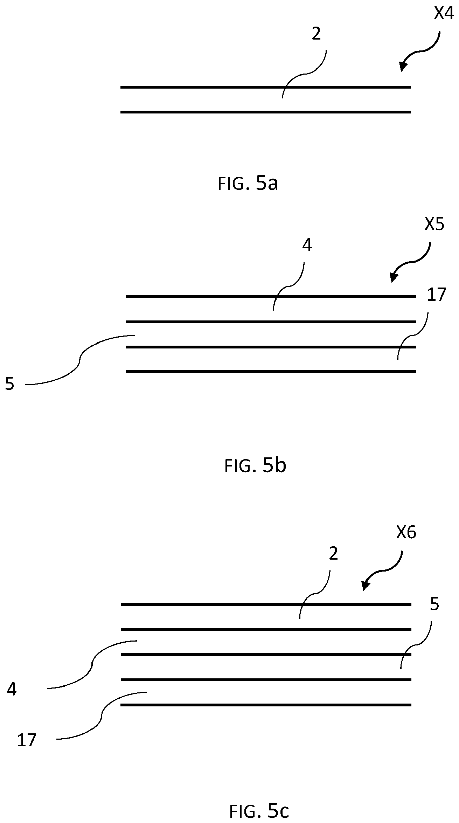

Fig. 5a, 5b, and 5c shows the make-up of an alternative embodiment of the packaging material and its constituents.

View X4 shows a top layer consisting of a porous HDPE film 2.

View X5 (not to scale) shows a laminate composed of a heat sealing PE film 4 at a thickness of 50 µm, an adhesive layer 5 (3 g/m²), and a film of oriented polyethylene (OPE) 17 at a thickness of 25 µm.

In view X6, the structure of the final packaging material, when the top layer has been sealed to the laminate, is shown. As will appear, the heat sealing PE film 4 faces the overlying layer of porous HDPE film 2.

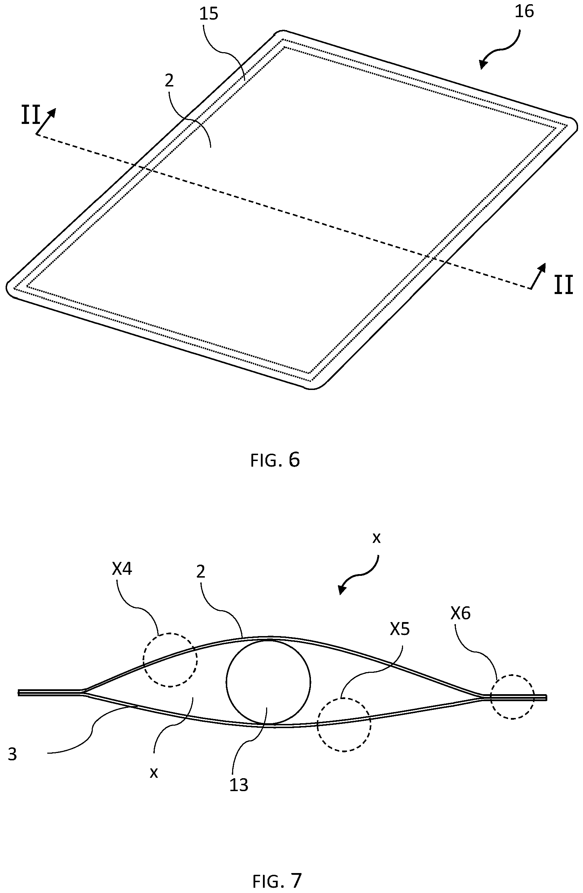

In Figs. 6 and 7, a bag prepared from the packaging material of Fig. 5 is depicted. The bag has a spacing 9 and contains a medical device 13. The bag is produced by superimposing the top layer on the laminate , so that the porous HDPE film 2 contacts the heat sealing PE film 4, applying heat in the regions to be sealed at a temperature of 80 °C at the side of the porous HDPE film 2 and at a temperature of 140 °C at the side of the OPE film for a period of 1 second.

### Example

### Test of laminates for peeling against porous HDPE film

Initially, 4 different 50 µm PE sealing films (Bjert 8449.050, Polifilm 2931.AAH.135-P, Polifilm 1226.AGM.134.wvn, and Polifilm 1424.AGM.134-P) were tested for their sealability against the porous HDPE film Tyvek 2FS.

Based on the results, two of the sealing films, namely Polifilm 2931.AAH.135-P (A) and Polifilm 1226.AGM.134.wvn (B), were selected for laminations trials done in a pilot plant and afterwards in a production line.

Corona-treated faces of A4-size sheets of the two selected sealing films A and B were laminated by adhesive lamination to corresponding corona-treated faces of A4-size sheets of a 25 µm OPE film (Jindal 25 HD200) by means of 3 g/m² of the 2-component adhesive Loctite Liofol LA 3644-21 MHS / LA 6055 from Henkel, resulting in the laminates A) and B), respectively.

The sealing film faces of the obtained laminates were sealed to the porous HDPE film Tyvek 1073B with a pair of sealing jaws. In one trial, the jaw pressing against the Tyvek side was not heated, whereas the jaw pressing against the OPE side of the laminate was heated to 125 °C. In a second trial, the jaw pressing against the Tyvek side was heated to 80 °C, whereas the jaw pressing against the OPE side of the laminate was heated to 125 °C. Sealing times of 1.0 or 2.0 seconds were employed.

The sealing strength of the resulting packaging material films were qualitatively assessed. The results are given in the below table

| Material | Sealing strength | | | |
|---|---|---|---|---|
| | Jaw 125 °C / non-heated jaw | | Jaw 125 °C / jaw 80 °C | |
| | 1.0 sec | 2.0 sec | 1.0 sec | 2.0 sec |
| A) | Not fully sealed | Satisfactory | Satisfactory | Not tested |
| B) | Sealing failed | Sealing failed | Satisfactory | Satisfactory |

Thus, when applying heat also from the Tyvek side of the material, an initial sealing temperature low enough to give an acceptable peelable sealing at 125 °C and a sealing time of 1 second was found for both of the tested materials.

In production trials it was found that only sealing film A is usable, when the laminate is used as a bottom web and the heat sealing is done through the Tyvek film. When sealing is done from the laminate film side, both sealing films A and B are usable, since the temperature of the sealing jaw on the laminate side can be increased to approximately 140 °C.

## Claims

1. A packaging material for allowing sterilization of contents of a package comprising packaging, such as a bag or tray, manufactured from the packaging material, wherein the packaging material comprises, consists of, or essentially consists of
a porous film comprising, consisting of, or essentially consisting of a high density polyethylene,
wherein the porous film shows a microbial barrier log retention value of 3 or more according to ASTM F1608 and a Bendtsen air permeability of 600 ml/min or more according to ISO 5636-3, and/or wherein the high density polyethylene comprises, consists of, or essentially consists of virgin filaments, the virgin filaments being flashspun and bonded to each other using heat and pressure with essentially no binders or fillers having been added, and
a laminate comprising a heat sealing polyethylene film adhered to either an oriented polyethylene film or to a layer of a high density polyethylene,
wherein at least a region of said heat sealing polyethylene film has been heat sealed to at least a region of said porous film.

2. A packaging material according to claim 1, wherein the heat sealing polyethylene film has a thickness of 15-200 µm and is optionally a low density polyethylene film.

3. A packaging material according to claim 1 or 2, wherein the oriented polyethylene film is a mono- or biaxially oriented film and has a thickness of 8-100 µm.

4. A packaging material according to any one of the preceding claims, wherein the heat sealing polyethylene film is adhered to either the oriented polyethylene film or to the layer of a high density polyethylene by means of an adhesive layer, which comprises, consists of, or essentially consists of an adhesive, and wherein the heat sealing polyethylene film has been adhesive laminated to the oriented polyethylene film or to the layer of a high density polyethylene by means of the adhesive layer, so that the heat sealing polymer film adheres to the oriented polyethylene film or to the layer of a high density polyethylene.

5. A packaging material according to claim 4, wherein the adhesive is a 2-component adhesive, and/or wherein the adhesive is applied in a grammage of 1-10 g/m³, 1-7 g/m³, or 2-3 g/m³.

6. A packaging material according to any one of the preceding claims, wherein the laminate comprises said layer of high density polyethylene and a further adhesive layer facing the layer of high density polyethylene, and an oriented polyethylene film facing the further adhesive layer.

7. A packaging material according to any one of the preceding claims, wherein the laminate is in the form of a tray, optionally thermoformed, comprising a bottom wall and a number of side walls defining a spacing for an item to be packaged, and wherein the porous film is in the form of a lid for sealing to an upper edge of the tray.

8. A packaging material according to claim 7, wherein the heat sealing polyethylene film has a thickness of 50-200 µm, 100-195 µm, 150-190 µm, or 170-185 µm, and/or the oriented polyethylene film has a thickness of 25-100 µm, 50-97 µm, 70-95 µm, or 80-90 µm.

9. A packaging material according to claim 7 or 8, wherein the tray comprises said layer of high density polyethylene, wherein the high density polyethylene is an expanded high density polyethylene, wherein the high density polyethylene is potentially rigid.

10. A packaging material according to any one of the preceding claims 7-9, wherein the heat sealing polyethylene film has a thickness of 15-80 µm, 30-70 µm, or 40-60 µm and/or the oriented polyethylene film has a thickness of 8-50 µm, 15-40 µm, 20-30 µm, or 24-26 µm.

11. A packaging material according to any one of claims 1 to 6, wherein the laminate comprises the oriented polyethylene film, the heat sealing polyethylene film of the packaging material sealing to rims of the porous film, such as to constitute a packaging in the form of a bag, wherein packaged contents, such as a medical device, is potentially positioned between the porous film and the laminate, wherein the laminate is potentially a flexible laminate.

12. A packaging, such as a bag or tray, for the manufacture of a package, wherein the packaging comprises, consists of, essentially consists of, or is manufactured from a packaging material according to any one of claims 1-11.

13. A package comprising a packaging according to claim 13, wherein contents, such as a medical device, are packed in the packaging.

14. Use of a package according to claim 12 for manufacture of a sterilized package, such as package comprising a medical device, the package being sterilized, such as by ethylene oxide sterilization, water vapour sterilization, gamma radiation sterilization, and/or plasma sterilization.

15. Use of a laminate for manufacture of a packaging material according to any one of claims 1-11, wherein the laminate comprises a heat sealing polyethylene film adhered to either an oriented polyethylene film or to a layer of a high density polyethylene.

16. A method of manufacture of a packaging material for allowing sterilization of contents of a package comprising packaging, such as a bag or tray, manufactured from the packaging material, wherein the packaging material is optionally according to any one of claims 1-11, wherein said method comprises, consists of, or essentially consists of
providing a porous film comprising, consisting of, or essentially consisting of a high density polyethylene, wherein the pourous film shows a microbial barrier log retention value of 3 or more according to ASTM F1608 and a Bendtsen air permeability of 600 ml/min or more according to ISO 5636-3, and/or wherein the high density polyethylene comprises, consists of, or essentially consists of virgin filaments, the virgin filaments being flashspun and bonded to each other using heat and pressure with essentially no binders or fillers having been added,
providing a laminate comprising a heat sealing polyethylene film adhered to either an oriented polyethylene film or to a layer of a high density polyethylene, such as by adhering the heat sealing polyethylene film to either the oriented polyethylene film or to the layer of the high density polyethylene, and
heat sealing at least a region of said heat sealing polyethylene film to at least a region of said porous film, such as by exposing the heat sealing polyethylene film to a sealing temperature of 80-160 °C, 85-140 °C, or 90-125 °C, for 0.5-3.0 seconds, 0.7-2.5 seconds, 0.8-2.0 seconds, or 0.9-1.0 seconds.
